(19) Europäisches Patentamt

European Patent Office

Office européen des brevets

(11) **EP 0 894 013 B1**

(12) **EUROPÄISCHE PATENTSCHRIFT**

(45) Veröffentlichungstag und Bekanntmachung des Hinweises auf die Patenterteilung:
**07.01.2004 Patentblatt 2004/02**

(51) Int Cl.[7]: **A61M 1/16**

(21) Anmeldenummer: **98905330.1**

(22) Anmeldetag: **22.01.1998**

(86) Internationale Anmeldenummer:
**PCT/EP1998/000326**

(87) Internationale Veröffentlichungsnummer:
**WO 1998/032476 (30.07.1998 Gazette 1998/30)**

(54) **VERFAHREN ZUR BESTIMMUNG VON PARAMETERN DER HÄMODIALYSE UND VORRICHTUNG ZUR DURCHFÜHRUNG DES VERFAHRENS**

PROCESS AND DEVICE FOR DETERMINING HEMODIALYSIS PARAMETERS

PROCEDE ET DISPOSITIF POUR DETERMINER DES PARAMETRES D'HEMODIALYSE

(84) Benannte Vertragsstaaten:
**DE ES FR GB IT**

(30) Priorität: **24.01.1997 DE 19702442**

(43) Veröffentlichungstag der Anmeldung:
**03.02.1999 Patentblatt 1999/05**

(73) Patentinhaber: **Fresenius Medical Care Deutschland GmbH**
**61352 Bad Homburg v.d.H. (DE)**

(72) Erfinder: **GOLDAU, Rainer**
**D-23847 Kastorf (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**EP-A- 0 291 421      EP-A- 0 428 927**
**EP-A- 0 528 437      EP-A- 0 532 433**
**EP-A- 0 547 025      EP-A- 0 658 352**

**Beschreibung**

Die Erfindung betrifft ein Verfahren zur Bestimmung von Parametern der Hämodialyse während einer extrakorporalen Blutbehandlung sowie eine Vorrichtung zur Durchführung des Verfahrens.

Eine wesentliche Aufgabe der Nieren des Menschen liegt in der Absonderung harnpflichtiger Stoffe aus dem Blut und der Regelung der Wasser- und Elektrolyt-Ausscheidung. Die Hämodialyse stellt ein Behandlungsverfahren zur Kompensation von Fehlfunktionen der Nieren bezüglich der Entfernung der harnpflichtigen Stoffe und der Einstellung der Elektrolyt-Konzentration im Blut dar.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration ($c_d$) der Dialysierflüssigkeit entspricht der Konzentration des Blutes eines Gesunden. Während der Behandlung wird das Blut des Patienten und die Dialysierflüssigkeit an beiden Seiten der Membran im allgemeinen im Gegenstrom mit einer vorgegebenen Flußrate ($Q_b$ bzw. $Q_d$) vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Durch Anlegen eines Transmembrandrucks kann der Stoffwechsel zusätzlich beeinflußt werden.

Um das Blutbehandlungsverfahren optimieren zu können, ist die Bestimmung von Parametern der Hämodialyse während der extrakorporalen Blutbehandlung (in-vivo) notwendig. Von Interesse ist insbesondere der Wert für die Austauschleistung des Dialysators, die durch die sogenannte "Clearance" bzw. "Dialysance D" dargestellt wird.

Als Clearance für einen bestimmten Stoff K wird dasjenige virtuelle (errechnete) Blutvolumen bezeichnet, das pro Minute unter definierten Bedingungen im Dialysator vollkommen von einem bestimmten Stoff befreit wird. Die Dialysance ist ein weiterer Begriff zur Bestimmung der Leistungsfähigkeit eines Dialysators, bei dem die Konzentration der eliminierten Substanz in der Dialysierflüssigkeit berücksichtigt wird. Neben diesen Parametern zur Beschreibung der Leistungsfähigkeit des Dialysators sind noch andere Parameter von Bedeutung, wie die Werte des wäßrigen Anteils des Blutes, des Blutvolumens und der Bluteingangskonzentration etc..

Die meßtechnisch-mathematische Quantifizierung der Blutreinigungsverfahren und die Bestimmung der vorgenannten Parameter der Dialyse ist relativ komplex. Hinsichtlich der Berechnungsgrundlagen wird auf Sargent,, J. A., Gotch. F.A.,: Principles and biophysics of dialysis, in: Replacement of Renal Function by Dialysis, W. Drukker, F. M. Parsons, J.F. Maher (Hrsg). Nijhoff, Den Haag 1983 verwiesen.

Die Dialysance bzw. die Clearance kann für einen gegebenen Elektrolyten, beispielsweise Natrium, bei einer Ultrafiltrationsrate von Null wie folgt bestimmt werden. Die Dialysance D ist gleich dem Verhältnis zwischen dem blutseitigen Massentransport für diesen Eletkrolyten ($Q_b$ x (cbi - cbo)) und der Konzentrationsdifferenz dieses Elektrolyten zwischen dem Blut und der Dialysierflüssigkeit am jeweiligen Eingang des Dialysators (cbi - cdi).

$$D = Qb \cdot \frac{cbi\text{-}cbo}{cbi\text{-}cdi} \tag{1}$$

Aus Gründen der Massenbilanz gilt:

$$Qb \cdot (cbi - cbo) = - Qd \cdot (cdi - cdo) \tag{2}$$

Aus den beiden oben genannten Gleichungen (1) und (2) folgt:

$$D = Qd \cdot \frac{cdi\text{-} cdo}{cbi\text{-}cdi} \tag{3}$$

Dabei bedeuten in (1) bis (3):

$Q_b$ =    effektiver Blutfluß
$Q_d$ =    Dialysierflüssigkeitsfluß
cb =    Stoffkonzentration im Blut
cd =    Stoffkonzentration in der Dialysierflüssigkeit
i =    Eingang des Dialysators
o =    Ausgang des Dialysators

Der effektive Blutfluß ist der Fluß des Blutanteils, in dem die zu entfernenden Stoffe gelöst sind, d.h., er bezieht sich auf das (wäßrige) Lösungsvolumen für diesen Stoff. Je nach Stoff kann das der Plasmawasserfluß oder der Blutwasserfluß, d.h. der gesamte Wasseranteil im Vollblut sein.

Die bekannten Verfahren zur in-vivo-Bestimmung von Parametern der Hämodialyse basieren auf den obigen Überlegungen. Dabei besteht das Bestreben, ohne einen direkten Meßeingriff in die Blutseite auszukommen, da dieser nämlich eine nicht unerhebliche Gefahrenquelle darstellt. Die zu bestimmenden Größen müssen daher allein aus dialysatseitigen Messungen abgeleitet werden.

Die DE 39 38 662 C2 (EP 0 428 927 A1) beschreibt ein Verfahren zur in-vivo-Bestimmung von Parametern der Hämodialyse, bei dem der Dialysat-Elektrolyttransfer jeweils bei zwei unterschiedlichen Dialysateingangskonzentrationen gemessen wird. Unter der Annahme, daß die Bluteingangskonzentration konstant ist, wird nach dem bekannten Verfahren die Dialysance dadurch bestimmt, daß die Differenz zwischen den Differenzen der Dialysierflüssigkeits-Ionenkonzentration an der Eingangsseite und der Ausgangsseite des Dialysators zum Zeitpunkt der ersten und zweiten Messung bestimmt wird, diese durch die Differenz der Dialysierflüssigkeits-Ionenkonzentration an der Eingangsseite zum Zeitpunkt der ersten Messung und der zweiten Messung geteilt wird und mit dem Dialysierflüssigkeitsfluß multipliziert wird. Bei diesem Verfahren erweist sich die verhältnismäßig lange Meßzeit als nachteilig, die darauf zurückzuführen ist, daß nach dem Einstellen der Dialysierflüssigkeit auf den neuen Eingangskonzentrationswert sich am Dialysatorausgang erst ein stabiler Gleichgewichtszustand einstellen muß, bevor der neue Meßwert aufgenommen werden kann. Es dauert systembedingt einen gewissen Zeitraum, bis ein Leitfähigkeitssprung am Dialysatoreingang zu stabilen Verhältnissen am Dialysatorausgang führt.

In dem Aufsatz von Niels A. Lassen, Ole Henriksen, Per Sejrsen in Handbook of Physiology, The Cardiovascular System, Vol. 3, Peripheral Circulation and Organ Blood Flow, Part I, American Physiological Society, 1983, wird die Bolusantwort eines intrakorporalen Kreislaufs auf eine Injektion und eine anschließende Messung der Konzentration näher behandelt, wobei Fragen der Signalfaltung eine wichtige Rolle spielen.

Der Erfindung liegt die Aufgabe zugrunde, ein Verfahren anzugeben, das eine schnelle Bestimmung von Parametern der Hämodialyse während einer extrakorporalen Blutbehandlung erlaubt. Darüber hinaus liegt der Erfindung die Aufgabe zugrunde, eine Vorrichtung zur Durchführung des Verfahrens zu schaffen.

Die Lösung dieser Aufgabe erfolgt erfindungsgemäß mit den im Patentanspruch 1 bzw. 8 angegebenen Merkmalen.

Bei dem erfindungsgemäßen Verfahren wird eine physikalische oder chemische Kenngröße der Dialysierflüssigkeit, z.B. die Stoffkonzentration in der Dialysierflüssigkeit zur Bestimmung der Dialysance D, im Dialysierflüssigkeitsweg stromauf des Dialysators verändert und die Kenngröße wird stromab des Dialysators gemessen. Die physikalische oder chemische Kenngröße sollte dabei auf physiologisch vertretbare Werte eingestellt werden. Sofern die Änderung der Kenngröße stromauf des Dialysators in ihrem zeitlichen Verlauf nicht bekannt ist, wird die Kenngröße auch stromauf des Dialysators gemessen. Zu den veränderbaren physikalischen oder chemischen Kenngrößen zählen auch die Dichte, der Brechungsindex, die Leitfähigkeit, die Temperatur oder die Dialysierflüssigkeitsrate.

Mit dem erfindungsgemäßen Verfahren kann ein Parameter der Hämodialyse, z.B. die Dialysance D, auch dann bestimmt werden, wenn sich ein Gleichgewichtszustand noch nicht eingestellt hat. Daher sind nur kurze Meßzeiten erforderlich. Da es bei längeren Meßzeiten, die bei den bekannten Verfahren zur Bestimmung von hämodynamischen Parametern erforderlich sind, zu einer systematischen Veränderung der hämodynamischen Parameter während der Messung kommen kann, sind kurze Meßzeiten von Vorteil. Darüber hinaus sind mit dem erfindungsgemäßen Verfahren auch kontinuierliche Messungen möglich.

Das erfindungsgemäße Verfahren beruht auf der Bestimmung der Antwort des Dialysators auf einen Diracstoß als Eingangssignal (Stoßantwort) aus dem zeitlichen Verlauf der physikalischen oder chemischen Kenngröße der Dialysierflüssigkeit stromauf und dem zeitlichen Verlauf der physikalischen oder chemischen Kenngröße stromab des Dialysators. Der hämodynamische Parameter wird dann aus der Stoßantwort des Dialysators bestimmt.

Die Stoßantwort des Dialysators kann bei einem beliebigen zeitlichen Verlauf der physikalischen oder chemischen Kenngröße stromauf oder stromab des Dialysators bestimmt werden. Prinzipiell ist es aber auch möglich, die Stoßantwort mit hinreichender Genauigkeit dadurch zu bestimmen, daß bei einem genügend scharfen Eingangsimpuls am Dialysatoreingang der zeitliche Verlauf der physikalischen oder chemischen Kenngröße stromab des Dialysators gemessen wird.

In einer bevorzugten Ausgestaltung der Erfindung wird zur Bestimmung der Dialysance als hämodynamischer Parameter das Integral über die Stoßantwort des Dialysators bestimmt. Das Integral über die Stoßantwort wird dann mit vorbestimmten Werten verglichen, die für eine bestimmte Dialysance charakteristisch sind. Die vorbestimmten Werte können beispielsweise nach dem aus der DE 39 38 662 C2 (EP 0 428 927 A1) bekannten Verfahren ermittelt werden.

In einer weiteren bevorzugten Ausgestaltung der Erfindung wird bei Kenntnis der Stoßantwort des Dialysators zu jedem Ausgangswert ein effektiver Meßwert errechnet, der bei einem stationären Gleichgewichtszustand am Dialysatoreingang gemessen worden wäre.

Bei dem erfindungsgemäßen Verfahren wird das dynamische System rechnerisch zu jedem Zeitpunkt wie das äquivalente stationäre System behandelt. Somit können die für die Bestimmung der Parameter der Hämodialyse erforderlichen Kenngrößen der Dialysierflüssigkeit auch dann ermittelt werden, wenn sich ein stationärer Zustand noch nicht eingestellt hat.

Nachfolgend werden das erfindungsgemäße Verfahren sowie eine Vorrichtung zur Durchführung des Verfahrens unter Bezugnahme auf die Figuren näher erläutert.

Es zeigen:

Fig. 1 ein Prinzipschaltbild der erfindungsgemäßen Vorrichtung, mit der das erfindungsgemäße Verfahren zur in-vivo-Bestimmung von Parametern der Hämodialyse durchgeführt wird,

Fig. 2a ein Gaußsignal als Beispielfunktion für eine Veränderung der Dialysierflüssigkeitseingangskonzentration und

Fig. 2b die Antwort des Dialysators auf das Gaußsignal von Fig. 2a.

Die erfindungsgemäße Vorrichtung zur Bestimmung von Parametern der Hämodialyse kann eine separate Baugruppe bilden. Sie kann aber auch Bestandteil einer Dialysevorrichtung sein, zumal einige Komponenten der erfindungsgemäßen Vorrichtung in den bekannten Dialysevorrichtungen bereits vorhanden sind. Nachfolgend wird die erfindungsgemäße Vorrichtung zusammen mit den wesentlichen Komponenten der Dialysevorrichtung beschrieben.

Die Dialysevorrichtung besteht im wesentlichen aus einem Dialysierflüssigkeitsteil 1 und einem extrakorporalen Blutkreislauf 2. Der extrakorporale Kreislauf umfaßt einen arteriellen Zweig 3, die Blutkammer 4 des Dialysators 5 und einen venösen Zweig 6. Im arteriellen Zweig 3 ist eine Blutpumpe 7 angeordnet, die einen bestimmten Blutfluß im extrakorporalen Kreislauf vorgibt.

Die durch eine semipermeable Membran 8 von der Blutkammer 4 getrennte Dialysierflüssigkeitskammer 9 des Dialysators ist über eine Zuführleitung 10 mit einer Dialysierflüssigkeitsquelle 11 verbunden. Eine Abführleitung 12, in die eine die Flußrate der Dialysierflüssigkeit vorgebende Dialysierflüssigkeitspumpe 13 geschaltet ist, führt in einen Abfluß 14.

In der Zuführleitung 10 und der Abführleitung 12 ist jeweils eine Meßeinrichtung 15, 16 zur Bestimmung der Ionenkonzentration der Dialysierflüssigkeit am Eingang des Dialysators 5 und der Stoffkonzentration der Dialysierflüssigkeit am Ausgang des Dialysators angeordnet. Die Meßeinrichtungen 15, 16 zur Bestimmung der Dialysierflüssigkeitseingangs- und -ausgangskonzentration weisen stromauf und stromab des Dialysators 8 angeordnete Leitfähigkeitssensoren auf, die vorzugsweise die temperaturkorrigierte Leitfähigkeit der Dialysierflüssigkeit auf der Basis der Na-Konzentration messen. Anstelle von Leitfähigkeitssensoren können auch optische Sensoren zur Messung der Dialysierflüssigkeitseingangs- bzw. -ausgangskonzentration im Dialysierflüssigkeitsweg angeordnet sein. Die Leitfähigkeitssensoren sind über Datenleitungen 17, 18 mit einer Speichereinheit 19 verbunden. Die Speichereinheit 19 empfängt die Meßwerte der Sensoren und speichert diese in zeitlicher Abfolge ab. Über eine Datenleitung 20 werden die Meßwerte einer Rechen- und Auswerteeinheit 21 zugeführt, die in einem Mikroprozessor aus den gewonnenen Daten die Parameter der Hämodialyse bestimmt. Ein derartiger Mikroprozessor ist in der Regel in einem Dialysegerät bereits vorhanden.

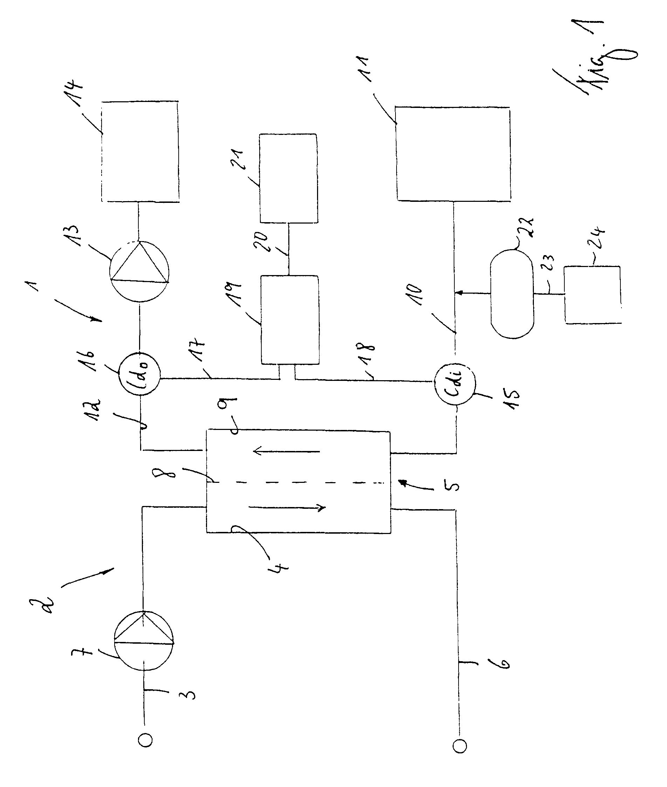

Zur Veränderung der Na-Konzentration der Dialysierflüssigkeit im Dialysierflüssigkeitsweg stromauf des Dialysators 5 ist eine Einrichtung 22 vorgesehen, die in Fig. 1 nur schematisch dargestellt ist. Mit der Einrichtung 22 kann der in den Dialysator fließenden Dialysierflüssigkeit ein Konzentratbolus aufgegeben werden. Der Ablauf der Messung wird von einer Steuereinheit 24 gesteuert, die über eine Signalleitung 23 mit der Einrichtung 22 zur Aufgabe eines Konzentratbolus verbunden ist.

Wenn die Bluttemperatur als hämodynamischer Parameter bestimmt werden soll, finden anstelle der Leitfähigkeitssensoren Temperatursensoren Verwendung, die im Dialysierflüssigkeitsweg stromauf bzw. stromab des Dialysators 5 angeordnet sind. Die Einrichtung 22 zur Veränderung der physikalischen oder chemischen Kenngröße im Dialysierflüssigkeitsweg stromauf des Dialysators ist in diesem Fall eine Heizeinrichtung zur Erzeugung eines Temperatursprungs.

Die Dialysierflüssigkeit durchströmt die Dialysierflüssigkeitskammer 9 mit einer durch die Drehzahl der Pumpe 13 vorgegebenen Flußrate $Q_d$ und der mittels der Einrichtung 22 veränderbaren Eingangskonzentration cdi, die mittels des stromauf angeordneten Leitfähigkeitssensors 15 erfaßt wird. Die sich bei der Dialyse einstellende Ausgangskonzentration cdo der Dialysierflüssigkeit wird mittels des stromab angeordneten Leitfähigkeitssensors 16 erfaßt.

Zum besseren Verständnis werden die theoretischen Grundlagen des Verfahrens zu Bestimmung der Hämodialyseparameter nachfolgend im einzelnen erläutert.

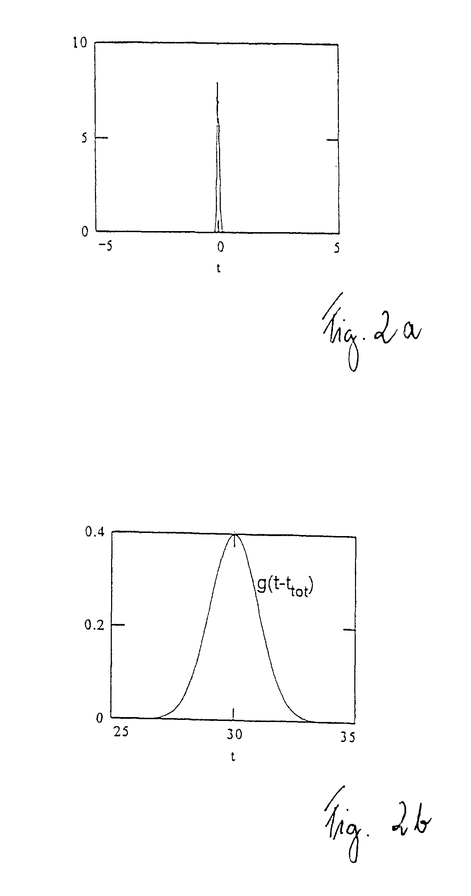

Fig. 2a zeigt ein Elektrolyt-Konzentrations-Bolus an der Eingangsseite des Dialysators, der zum Zeitpunkt t=0 appliziert wird. Seine Dauer ist klein gegenüber der Flußzeit der Dialysierflüssigkeit durch den Dialysator, wobei seine Grundlinie der vorgegebenen Soll-Konzentration des betrachteten Elektrolyts entspricht.

Der Konzentratbolus läßt sich durch eine Leitfähigkeitsmessung der Dialysierflüssigkeit stromauf und stromab des Dialysators nachweisen. Der am Dialysatoreingang angeordnete Leitfähigkeitssensor 15 mißt ein Flüssigkeitselement dV, das den Leitfähigkeitsimpuls trägt, als scharfen Impuls. Das Flüssigkeitselement dV gelangt dann durch das aus dem Schlauchzulauf, dem Dialysator und dem Schlauchablauf gebildete System zu dem stromab des Dialysators angeordneten Leitfähigkeitssensor 16. Das Maximum des Impulses benötigt dazu die Zeit $t_{tot}$, wobei der Impuls durch die unterschiedliche Laufzeit der Einzelteilchen verbreitert wird und nun die Form der in Fig. 2b dargestellten Funktion $g(t-t_{tot})$ hat.

Für die Beziehung zwischen dem zeitlichen Verlauf der Dialysierflüssigkeitsausgangskonzentration cdo(t) und der Dialysierflüssigkeitseingangskonzentration cdi(t) kann das folgende Faltungsintegral formuliert werden:

$$cdo(t) := \int_{t-t_g}^{t+t_g} cdi\left(t' - t_{tot}\right) \cdot f(t') \cdot g(t - t') \, dt' \qquad (4)$$

In dem obigen Faltungsintiegral ist $t_g$ die Zeitspanne, die eine Impulsantwort auf der Ausgangsseite benötigt, um von ihrem Maximum unter die halbe Meßgenauigkeit abzufallen oder anzusteigen. Für die Zeit $t_g$ gilt:

$$\frac{g(t_g)}{g(0)} \le \frac{M}{2} \qquad (5)$$

wobei M die geforderte Meßgenauigkeit ist.

Die Funktion f(t) ist ein zeitlich veränderlicher Korrekturfaktor, der den Teilchenverlust/-gewinn berücksichtigt. Unter der Annahme zeitlich konstanter Teilchenverluste oder -gewinne im Dialysator ist f(t) konstant. Für den Fall, daß es zu keinem Teilchenaustausch kommt, ist f(t) gleich 1.

Der zeitliche Verlauf der Dialysierflüssigkeitseingangs- und -ausgangskonzentration cdi(t) und cdo(t) kann durch die Leitfähigkeitssensoren stromauf und stromab des Dialysators ermittelt werden. Ebenso kann die Zeit $t_g$ gemessen werden. Die Stoßantwort kann über jeden beliebigen Eingangsbolus und eine anschließende Laplace-Transformation entfaltet werden. Das bedeutet insbesondere, daß keine vorherige, getrennte Messung zur Bestimmung der Stoßantwort erforderlich ist. Vielmehr kann die Stoßantwort direkt aus den Meßdaten abgeleitet werden. Die Stoßantwort g(t) kann aber auch durch eine vorausgehende Messung der Dialysierflüssigkeitsausgangskonzentration ermittelt werden, wenn auf die in den Dialysator fließende Dialysierflüssigkeit mittels der Einrichtung 22 ein sehr schmaler (idealerweise infinitesimaler) Elektrolyt-Konzentrations-Bolus aufgegeben wird.

Die Zeit $t_{tot}$ kann über das Maximum der Korrelationsfunktion K der zeitlichen Verläufe von der Dialysierflüssigkeitseingangs- und ausgangskonzentration cdi(t) und cdo(t) bis zum Zeitpunkt t erhalten werden, wenn die Funktionen cdi(t) und cdo(t) zeitlich gegeneinander um die Zeit $t_v$ verschoben werden, wobei sich die Korrelationsfunktion $K(t_v)$ wie folgt berechnet.

$$K(t_v) = \frac{\int_0^t \left(cdi(t') - cdi_{mittel}\right) \cdot \left(cdo(t' + t_v) - cdo_{mittel}\right) dt'}{\sqrt{\int_0^t \left(cdi(t') - cdi_{mittel}\right)^2 dt' \cdot \int_0^t \left(cdo(t' + t_v) - cdo_{mittel}\right)^2 dt'}} \qquad (6)$$

wobei $cdi_{mittel}$ der Mittelwert aus allen cdi und $cdo_{mittel}$ der Mittelwert aus allen cdo ist.

Die Stoßantwort g(t) stellt den Kern des Faltungsintegrals (4) dar und kann daher als Wichtungsfunktion aufgefaßt werden, die angibt, welchen Anteil an der Dialysierflüssigkeitsausgangskonzentration cdo(t) zum Zeitpunkt t ein bestimmter Wert der Dialysierflüssigkeitseingangskonzentration $cdi(t'-t_{tot})$ zum Zeitpunkt $t'-t_{tot}$ besitzt, wobei $t'$ im Bereich von $(t-t_g, t+t_g)$ liegt. Diese Wichtung erlaubt es, jederzeit die zu einer bestimmten Dialysierflüssigkeitsausgangskonzentration gehörige Dialysierflüssigkeitseingangskonzentration zu ermitteln und entsprechend gewichtet zu einer äquivalenten Dialysierflüssigkeitseingangskonzentration umzurechnen, die geherrscht haben müßte, wenn das System stationär gewesen wäre.

Die einem stationären Zustand entsprechende Dialysierflüssigkeitseingangskonzentration $cdi_{stat}(t)$ ergibt sich zu

$$cdi_{stat}(t) = \int_{t-t_g}^{t+t_g} cdi(t'-t_{tot}) g(t-t') dt' \qquad (7)$$

Bei einem ersten Ausführungsbeispiel der unter Bezugnahme auf Fig. 1 beschriebenen Vorrichtung zur Bestimmung von Parametern der Hämodialyse, insbesondere der Dialysance D, wird der Meßablauf von der Steuereinheit wie folgt vorgegeben.

Die Steuereinheit 24 sendet ein Steuersignal an die Einrichtung 22 zur Veränderung der Dialysierflüssigkeitskonzentration, die daraufhin einen Konzentratbolus stromauf des Dialysators 5 erzeugt. Der zeitliche Verlauf der Änderung der Dialysierflüssigkeitseingangs- und ausgangskonzentration cdi, cdo wird mittels der Leitfähigkeitssensoren 15, 16 erfaßt, deren Meßwerte in der Speichereinheit 19 abgelegt werden. In der Rechen- und Auswerteeinheit 21 werden die durch eine Folge von diskreten Meßwerten dargestellten Eingangs- und Ausgangssignale analysiert, wobei $t_{tot}$ und $t_g$ nach den obigen Gleichungen berechnet werden. Aus der in einer vorausgehenden Messung ermittelten oder vorgegebenen Stoßantwort des Dialysators und den Werten der Dialysierflüssigkeitseingangskonzentration cdi werden nach Gleichung (7) die einem stationären Zustand entsprechenden Werte der Dialysierflüssigkeitseingangskonzentration $cdi_{stat}(t_1)$ und $cdi_{stat}(t_2)$ ermittelt. Die Dialysance wird dann in der Recheneinheit nach der folgenden Gleichung berechnet:

$$D = Q_d \frac{(cdi_{stat}(t_1) - cdo(t_1)) - (cdi_{stat}(t_2) - cdo(t_2))}{cdi_{stat}(t_1) - cdi_{stat}(t_2)} \qquad (8)$$

Bei einem zweiten Ausführungsbeispiel der unter Bezugnahme auf Figur 1 beschriebenen Vorrichtung wird aus dem zeitlichen Verlauf der Dialysierflüssigkeitseingangskonzentration cdi (t) und dem zeitlichen Verlauf der Dialysierflüssigkeitsausgangskonzentration cdo (t) in der Rechen- und Auswerteinheit 21 die Stoßantwort des Dialysators 5 durch eine Laplace-Transformation berechnet. Anschließend wird in der Rechen- und Auswerteinheit das Integral über die Stoßantwort des Dialysators berechnet. Hierzu verfügt die Auswert- und Recheneinheit über einen Integrator. Die Auswert- und Recheneinheit umfaßt darüber hinaus einen Speicher, in dem eine (vorher kalibrierte) Zuordnungstabelle abgelegt ist. Die Tabelle ordnet für verschiedene Dialysierflüssigkeitsflußraten Qd verschiedenen Werten des Integrals über die Stoßantwort des Dialysators eine bestimmte Dialysance zu. Die Stoßantwort T(t, t') des Dialysators wird in Gleichung (4) durch das Produkt f(t')g(t-t') wiedergegeben. Die Rechen- und Auswerteinheit 21 vergleicht das Integral über die aus der Dialysierflüssigkeitseingangs- und Ausgangskonzentration berechneten Stoßantwort des Dialysators mit den im Speicher abgelegten Werten und bestimmt dann die Dialysance des Dialysators.

Mit dem erfindungsgemäßen Verfahren kann nicht nur die Dialysance ermittelt werden, sondern es ist auch möglich, weitere Parameter der Hämodialyse, z.B. die Bluteingangskonzentration, der effektive Blutwasserfluß und der Hämatokrit in einer kontinuierlichen Messung während der Dialyse zu bestimmen. Die Vorteile, die sich aus der Berechnung einer Dialysierflüssigkeitseingangskonzentration, die einem stationären Zustand entspricht, ergeben, kommen bei allen Meßverfahren zur Bestimmung von Parametern der Hämodialyse zum tragen, bei denen physikalische oder chemische Kenngrößen, z.B. die Stoffkonzentration der Dialysierflüssigkeit, im Dialysierflüssigkeitsweg stromauf und stromab des Dialysators gemessen werden.

**Patentansprüche**

**1.** Verfahren zur Bestimmung von Parametern der Hämodialyse während einer extrakorporalen Blutbehandlung, bei der das zu behandelnde Blut in einem extrakorporalen Kreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitsweg die Dialysierflüssigkeitskammer des Dialysators durchströmt, mit folgenden Verfahrensschritten:

- eine physikalische oder chemische Kenngröße cdi der Dialysierflüssigkeit wird im Dialysierflüssigkeitsweg stromauf des Dialysators verändert und die physikalische oder chemische Kenngröße cdo der Dialysierflüssigkeit wird stromab des Dialysators gemessen und

- aus der physikalischen oder chemischen Kenngröße der Dialysierflüssigkeit stromauf und stromab des Dialysators wird der Parameter der Hämodialyse bestimmt,

wobei der zeitliche Verlauf der physikalischen oder chemischen Kenngröße der Dialysierflüssigkeit stromauf des Dialysators ein Eingangssignal des Dialysators und der zeitliche Verlauf der physikalischen oder chemischen Kenngröße der Dialysierflüssigkeit stromab des Dialysators die Antwort des Dialysators auf das Eingangssignal beschreibt,
**dadurch gekennzeichnet,**
**daß** aus dem zeitlichen Verlauf der physikalischen oder chemischen Kenngröße der Dialysierflüssigkeit stromauf und dem zeitlichen Verlauf der physikalischen oder chemischen Kenngröße der Dialysierflüssigkeit stromab des Dialysators die Antwort des Dialysators auf einen Diracstoß als Eingangssignal (Stoßantwort) bestimmt wird und die Stoßantwort des Dialysators zur Bestimmung der Parameter der Hämodialyse herangezogen wird.

2. Verfahren nach Anspruch 1, **dadurch gekennzeichnet, daß** das Integral über die Stoßantwort des Dialysators bestimmt wird und daß zur Bestimmung der Dialysance als hämodynamischer Parameter das Integral über die Stoßantwort mit vorbestimmten Werten verglichen wird, die für eine bestimmte Dialysance charakteristisch sind.

3. Verfahren nach Anspruch 1, **dadurch gekennzeichnet, daß** aus der Stoßantwort des Dialysators und den Werten der physikalischen oder chemischen Kenngröße cdi der Dialysierflüssigkeit stromauf des Dialysators die einem stationären Zustand entsprechenden Werte $cdi_{stat}$ der physikalischen oder chemischen Kenngröße der Dialysierflüssigkeit stromauf des Dialysators ermittelt werden, und aus den einem stationären Zustand entsprechenden Werten der physikalischen oder chemischen Kenngröße der Dialysierflüssigkeit der Parameter der Hämodialyse bestimmt wird.

4. Verfahren nach einem der Ansprüche 1 bis 3, **dadurch gekennzeichnet, daß** die physikalische oder chemische Kenngröße der Dialysierflüssigkeit stromauf bzw. stromab des Dialysators die Stoffkonzentration in der Dialysierflüssigkeit stromauf des Dialysators (Dialysierflüssigkeitseingangskonzentration cdi) bzw. stromab des Dialysators (Dialysierflüssigkeitsausgangskonzentration cdo) ist.

5. Verfahren nach einem der Ansprüche 1 bis 4, **dadurch gekennzeichnet, daß** zur Bestimmung der Stoffkonzentration die Leitfähigkeit der Dialysierflüssigkeit gemessen wird.

6. Verfahren nach Anspruch 4 oder 5, **dadurch gekennzeichnet, daß** die Dialysierflüssigkeitseingangskonzentration cdi in einem vorgegebenen Zeitintervall verändert wird und daß zur Bestimmung der Dialysance D als Parameter der Dialyse die Differenz zwischen den Differenzen der einem stationären Zustand entsprechenden Dialysierflüssigkeitseingangskonzentration $cdi_{stat}$ und der Dialysierflüssigkeitsausgangskonzentration cdo zum Zeitpunkt einer ersten und einer nachfolgenden zweiten Messung bestimmt wird, durch die Differenz der einem stationären Zustand entsprechenden Dialysierflüssigkeitseingangskonzentration zum Zeitpunkt der ersten Messung und der Dialysierflüssigkeitseingangskonzentration zum Zeitpunkt der zweiten Messung geteilt wird und mit dem Dialysierflüssigkeitsfluß $Q_d$ multipliziert wird.

7. Verfahren nach einem der Ansprüche 4 bis 6, **dadurch gekennzeichnet, daß** die einem stationären Zustand entsprechenden Werte der Dialysierflüssigkeitseingangskonzentration aus der Stoßantwort des Dialysators und den Werten der physikalischen oder chemischen Kenngröße der Dialysierflüssigkeit stromauf des Dialysators nach der Beziehung

$$cdi_{stat}(t) = \int_{-t_g}^{+t_g} cdi(t'-t_{tot})g(t-t')dt' \qquad (7)$$

bestimmt werden, wobei $t_{tot}$ die mittlere Laufzeit des Eingangsimpulses zwischen dem Dialysatoreingang und dem Dialysatorausgang und tg die Zeitspanne ist, die eine Impulsanwort auf der Ausgangsseite benötigt, um von ihrem Maximum unter die halbe Meßgenauigkeit abzufallen oder anzusteigen.

8. Vorrichtung zur Durchführung des Verfahrens nach einem der Ansprüche 1 bis 7 für eine Blutbehandlungsvorrichtung, bei der in einem extrakorporalen Kreislauf die Blutkammer (4) eines durch eine semipermeable Membran (8) in die Blutkammer und eine Dialysierflüssigkeitskammer unterteilten Dialysators (5) von dem zu behandelnden Blut durchströmbar ist und in einem Dialysierflüssigkeitsweg die Dialysierflüssigkeitskammer (9) des Dialysators (5) von Dialysierflüssigkeit durchströmbar ist, wobei in den extrakorporalen Kreislauf eine Blutpumpe (7) und in den Dialysierflüssigkeitsweg eine Dialysierflüssigkeitspumpe (13) geschaltet ist, mit
einer Einrichtung (22) zum Verändern der physikalischen oder chemischen Kenngröße cdi der Dialysierflüssigkeit im Dialysierflüssigkeitsweg stromauf des Dialysators (5) in einem vorgegebenen Zeitintervall,
einer Meßeinrichtung (16) zur Erfassung der physikalischen oder chemischen Kenngröße cdo der Dialysierflüssigkeit im Dialysierflüssigkeitsweg stromab des Dialysators (5),
einer Speichereinheit (19), die derart ausgebildet ist, daß die gemessenen Werte der physikalischen oder chemischen Kenngröße cdo der Dialysierflüssigkeit stromab des Dialysators (5) in zeitlicher Abfolge abspeicherbar sind und
einer Rechen- und Auswerteeinheit (21), die derart ausgebildet ist, daß aus der Kenngröße cdi und der Kenngröße cdo der Parameter der Hämodialyse bestimmbar ist,
**dadurch gekennzeichnet,**
**daß** die Rechen- und Auswerteinheit (21) derart ausgebildet ist, daß aus dem zeitlichen Verlauf der physikalischen oder chemischen Kenngröße der Dialysierflüssigkeit stromauf und dem zeitlichen Verlauf der physikalischen oder chemischen Kenngröße der Dialysierflüssigkeit stromab des Dialysators (5) die Antwort des Dialysators auf einen Diracstoß als Eingangssignal (Stoßantwort) bestimmbar ist und der Parameter der Hämodialyse aus der Stoßantwort des Dialysators ermittelt wird.

9. Vorrichtung nach Anspruch 8, **dadurch gekennzeichnet, daß** die Rechenund Auswerteinheit (21) einen Integrator zur Bestimmung des Integrals über die Stoßantwort des Dialysators und einen Speicher umfaßt, in dem vorbestimmte Werte abgelegt sind, die für eine bestimmte Dialysance charakteristisch sind und daß die Rechen- und Auswerteinheit derart ausgebildet ist, daß durch den Vergleich des Integrals über die Stoßantwort mit den im Speicher abgelegten Werten die Dialysance als der hämodynamische Parameter bestimmbar ist.

10. Vorrichtung nach Anspruch 8, **dadurch gekennzeichnet, daß** aus der Stoßantwort des Dialysators (5) und den Werten der physikalischen oder chemischen Kenngröße cdi der Dialysierflüssigkeit stromauf des Dialysators die einem stationären Zustand entsprechenden Werte der physikalischen oder chemischen Kenngröße $cdi_{stat}$ der Dialysierflüssigkeit stromauf des Dialysators bestimmbar sind und der Parameter der Hämodialyse aus den einem stationären Zustand entsprechenden Werten der Kenngröße $cdi_{stat}$ und den abgespeicherten Werten der physikalischen oder chemischen Kenngröße cdo der Dialysierflüssigkeit stromab des Dialysators ermittelt wird.

11. Vorrichtung nach einem der Ansprüche 8 bis 10, **dadurch gekennzeichnet, daß** die Einrichtung (22) zum Verändern der physikalischen oder chemischen Kenngröße als eine Einrichtung zum Verändern der Stoffkonzentration in der Dialysierflüssigkeit stromauf des Dialysators (Dialysierflüssigkeitseingangskonzentration cdi) oder der Temperatur der Dialysierflüssigkeit und die Meßeinrichtung (16) zur Erfassung der physikalischen oder chemischen Kenngröße als eine Meßeinrichtung zur Erfassung der Stoffkonzentration in der Dialysierflüssigkeit stromab des Dialysators (Dialysierflüssigkeitsausgangskonzentration cdo) oder der Temperatur der Dialysierflüssigkeit ausgebildet sind.

12. Vorrichtung nach Anspruch 11, **dadurch gekennzeichnet, daß** die Meßeinrichtung (16) zur Erfassung der physikalischen oder chemischen Eigenschaft einen im Dialysierflüssigkeitsweg stromab des Dialysators angeordneten Leitfähigkeitssensor oder optischen Sensor zur Bestimmung der Dialysierflüssigkeitsausgangskonzentration cdo oder einen Temperatursensor zur Bestimmung der Dialysierflüssigkeitstemperatur aufweist.

**Claims**

1. Process for determining haemodialysis parameters during an extra-corporeal blood treatment, in which the blood to be treated flows in an extracorporeal circuit through the blood chamber of a dialyser subdivided by a semipermeable membrane into a blood chamber and a dialysis fluid chamber, and wherein dialysis fluid flows in a dialysis fluid path through the dialysis fluid chamber of the dialyser, with the following process stages:

   - a physical or chemical characteristic value cdi of the dialysis fluid is varied in the dialysis fluid path upstream of the dialyser and the physical or chemical characteristic value cdo of the dialysis fluid is measured downstream of the dialyser, and

   - the haemodialysis parameter is determined from the physical or chemical characteristic value of the dialysis upstream and downstream of the dialyser,

   wherein the variation in time of the physical or chemical characteristic value of the dialysis fluid upstream of the dialyser describes an input signal of the dialyser, and the variation in time of the physical or chemical characteristic value of the dialysis fluid downstream of the dialyser describes the response of the dialyser to the input signal, **characterised in that**
   the response of the dialyser to a Dirac pulse as input signal (pulse response) is determined from the variation in time of the physical or chemical characteristic value of the dialysis fluid upstream, and from the variation in time of the physical or chemical characteristic value of the dialysis fluid downstream of the dialyser, and **in that** the pulse response of the dialyser is used to determine the haemodialysis parameters.

2. Process according to daim 1, **characterised in that** the integral of the pulse response of the dialyser is determined and **in that** the pulse response integral is compared with predetermined values characteristic of a certain dialysance for determining the dialysance as a haemodynamic parameter.

3. Process according to daim 1, **characterised in that** the values $cdi_{stat}$ of the physical or chemical characteristic value of the dialysis fluid upstream of the dialyser are determined from the pulse response of the dialyser and the values of the physical or chemical characteristic value cdi of the dialysis fluid upstream of the dialyser, and **in that** the haemodialysis parameter is determined from the values of the physical or chemical characteristic value of the dialysis fluid of the dialysis fluid corresponding to a state of static equilibrium.

4. Process according to one of claims 1 to 3, **characterised in that** the physical or chemical characteristic value of the dialysis fluid upstream and downstream of the dialyser is the substance concentration in the dialysis fluid upstream of the dialyser (dialysis fluid inlet concentration cdi) and downstream of the dialyser (dialysis fluid outlet concentration cdo).

5. Process according to one of claims 1 to 4, **characterised in that** the conductivity of the dialysis fluid is measured to determine the substance concentration.

6. Process according to daim 4 or 5, **characterised in that** the dialysis fluid inlet concentration cdi is varied within a predetermined interval of time, and **in that** in order to determine the dialysance D as a dialysis parameter, the difference is determined between the differences of a dialysis fluid inlet concentration $cdi_{stat}$ corresponding to a state of static equilibrium and the dialysis fluid outlet concentration cdo at the time of a first, then a second measurement, divided by the difference between the dialysis fluid inlet concentration at the time of the first measurement and the dialysis fluid inlet concentration at the time of the second measurement, and multiplied by the dialysis fluid flow $Q_d$.

7. Process according to one of daims 4 to 6, **characterised in that** the values of the dialysis fluid inlet concentration corresponding to a state of static equilibrium are determined from the pulse response of the dialyser and the values of the physical or chemical characteristic value of the dialysis fluid upstream of the dialyser, according to the relation

$$cdi_{stat}(t) = \int_{-tg}^{t+tg} cdi(t^1-t_{tot})g(t-t_1)dt_1 \qquad (7)$$

wherein $t_{tot}$ is the mean running time of the inlet pulse between the dialyser inlet and the dialyser outlet, and $t_g$ is the period of time a pulse response requires on the outlet side to drop from its maximum to below half the measuring accuracy or to increase.

8. Device for carrying out the process according to one of claims 1 to 7 for a blood treatment device in which the blood to be treated is able to flow in an extracorporeal circuit through the blood chamber (4) of a dialyser (5) subdivided by a semipermeable membrane (8) into the blood chamber and a dialysis fluid chamber, and dialysis fluid is able to flow in a dialysis fluid path through the dialysis fluid chamber (9) of the dialyser (5), wherein a blood pump (7) is inserted in the extracorporeal circuit, and a dialysis fluid pump (13) is inserted in the dialysis fluid path, with

a device (22) for varying the physical or chemical characteristic value cdi of the dialysis fluid in the dialysis fluid path upstream of the dialyser (5) within a predetermined interval of time,

a measuring device (16) for recording the physical or chemical characteristic value cdo of the dialysis fluid in the dialysis fluid path downstream of the dialyser (5),

a storage unit (19) which is designed so that the measured values of the physical or chemical characteristic value cdo of the dialysis fluid downstream of the dialyser (5) can be stored in time sequence, and

a counting and evaluating unit (21) which is designed so that the haemodialysis parameter can be determined from the characteristic value cdi and the characteristic value cdo,

**characterised in that**

the counting and evaluating unit (21) is designed so that the response of the dialyser to a Dirac pulse can be determined as the inlet signal (pulse response) from the variation in time of the physical or chemical characteristic value of the dialysis fluid upstream, and the variation in time of the physical or chemical characteristic value of the dialysis fluid downstream of the dialyser (5), and **in that** the haemodialysis parameter is determined from the pulse response of the dialyser.

9. Device according to claim 8, **characterised in that** the counting and evaluating unit (21) comprises an integrator for determining the pulse response integral of the dialyser and a memory in which are stored predetermined values which are characteristic of a certain dialysance, and **in that** the counting and evaluating unit is designed so that the dialysance can be determined as the haemodynamic parameter by comparing the pulse response integral with the values stored in the memory.

10. Device according to claim 8, **characterised in that** the values of the physical or chemical characteristic value cdi$_{stat}$ of the dialysis fluid upstream of the dialyser, corresponding to a state of static equilibrium, can be determined from the pulse response of the dialyser (5) and the values of the physical or chemical characteristic value cdi of the dialysis fluid upstream of the dialyser, and **in that** the haemodialysis parameter is determined from the values of the characteristic value cdi$_{stat}$ corresponding to a state of static equilibrium and from the stored values of the physical or chemical characteristic value cdo of the dialysis fluid downstream of the dialyser.

11. Device according to one of claims 8 to 10, **characterised in that** the device (22) for varying the physical or chemical characteristic value is designed as a device for varying the substance concentration in the dialysis fluid upstream of the dialyser (dialysis fluid inlet concentration cdi) or the temperature of the dialysis fluid, and **in that** the measuring device (16) for recording the physical or chemical characteristic value is designed as a measuring device for recording the substance concentration in the dialysis fluid downstream of the dialyser (dialysis fluid outlet concentration cdo) or the temperature of the dialysis fluid.

12. Device according to claim 11, **characterised in that** the measuring device (16) for recording the physical or chemical property exhibits a conductivity sensor or optical sensor arranged in the dialysis fluid path downstream of the dialyser for determining the dialysis fluid outlet concentration cdo or a temperature sensor for determining the dialysis fluid temperature.

**Revendications**

1. Procédé pour déterminer des paramètres de l'hémodialyse pendant un traitement du sang extracorporel dans lequel le sang à traiter traverse dans un circuit extracorporel la chambre à sang d'un dialyseur divisé par une membrane semi-perméable en la chambre à sang et une chambre à liquide de dialyse, et le liquide de dialyse traverse dans une voie de liquide de dialyse la chambre à liquide de dialyse, avec les étapes de procédé suivantes :

   - une caractéristique physique ou chimique cdi du liquide de dialyse est modifiée dans la voie de liquide de dialyse en amont du dialyseur et la caractéristique physique ou chimique cdo du liquide de dialyse est mesurée en aval du dialyseur et
   - le paramètre de l'hémodialyse est déterminé d'après la caractéristique physique ou chimique du liquide de dialyse en amont et en aval du dialyseur,

   où l'évolution temporelle de la caractéristique physique ou chimique du liquide de dialyse en amont du dialyseur décrit un signal d'entrée du dialyseur et l'évolution temporelle de la caractéristique physique ou chimique du liquide de dialyse en aval du dialyseur décrit la réponse du dialyseur au signal d'entrée,
   **caractérisé en ce que** la réponse du dialyseur a une impulsion de Dirac comme signal d'entrée (réponse impulsionnelle) est déterminée d'après l'évolution temporelle de la caractéristique physique ou chimique du liquide de dialyse en amont et l'évolution temporelle de la caractéristique physique ou chimique du liquide de dialyse en aval du dialyseur, et la réponse impulsionnelle du dialyseur est utilisée pour déterminer les paramètres de l'hémodialyse.

2. Procédé selon la revendication 1 **caractérisé en ce que** l'intégrale de la réponse impulsionnelle du dialyseur est déterminée et **en ce que**, pour déterminer la dialysance comme paramètre hémodynamique, l'intégrale de la réponse impulsionnelle est comparée à des valeurs prédéterminées qui sont caractéristiques d'une dialysance déterminée.

3. Procédé selon la revendication 1 **caractérisé en ce que** les valeurs $cdi_{stat}$, correspondant à un état stationnaire, de la caractéristique physique ou chimique du liquide de dialyse en amont du dialyseur sont déterminées d'après la réponse impulsionnelle du dialyseur et d'après les valeurs de la caractéristique physique ou chimique cdi du liquide de dialyse en amont du dialyseur, et le paramètre de l'hémodialyse est déterminé d'après les valeurs de la caractéristique physique ou chimique du liquide de dialyse correspondant à un état stationnaire.

4. Procédé selon l'une des revendications 1 à 3 **caractérisé en ce que** la caractéristique physique ou chimique du liquide de dialyse en amont ou en aval du dialyseur est la concentration de substance dans le liquide de dialyse en amont du dialyseur (concentration d'entrée du liquide de dialyse cdi) ou en aval du dialyseur (concentration de sortie du liquide de dialyse cdo).

5. Procédé selon l'une des revendications 1 à 4 **caractérisé en ce que** la conductibilité du liquide de dialyse est mesurée pour déterminer ia concentration de substance.

6. Procédé selon la revendication 4 ou 5 **caractérisé en ce que** la concentration d'entrée du liquide de dialyse cdi est modifiée dans un intervalle de temps prédéterminé et **en ce que**, pour déterminer la dialysance D comme paramètre de la dialyse, la différence entre les différences de la concentration d'entrée du liquide de dialyse $cdi_{stat}$ correspondant à un état stationnaire et la concentration de sortie du liquide de dialyse cdo au moment d'une première mesure et d'une seconde mesure successives est déterminée, divisée par la différence de la concentration d'entrée du liquide de dialyse correspondant à un état stationnaire au moment de la première mesure et de la concentration d'entrée du liquide de dialyse au moment de la deuxième mesure et multipliée par le flux de liquide de dialyse $Q_d$.

7. Procédé selon l'une des revendications 4 à 6 **caractérisé en ce que** les valeurs de la concentration d'entrée du liquide de dialyse correspondant à un état stationnaire sont déterminées d'après la réponse impulsionnelle du dialyseur et d'après les valeurs de la caractéristique physique ou chimique du liquide de dialyse en amont du dialyseur selon la relation

$$cdi_{stat}(t) = \int_{-t_g}^{+t_g} cdi(t'-t_{tot})g(t-t')dt' \qquad (7)$$

où $t_{tot}$ est la durée moyenne de l'impulsion d'entrée entre l'entrée du dialyseur et la sortie du dialyseur et $t_g$ est la durée nécessaire à une réponse impulsionnelle du côté de la sortie pour diminuer ou augmenter depuis son maximum de moins de la moitié de la précision de la mesure.

8. Dispositif pour la mise en oeuvre du procédé selon l'une des revendications 1 à 7 pour un dispositif de traitement du sang dans lequel, dans un circuit extracorporel, la chambre à sang (4) d'un dialyseur (5) divisé par une membrane semi-perméable (8) en la chambre à sang et en une chambre à liquide de dialyse peut être traversée par le sang à traiter et, dans une voie de liquide de dialyse, la chambre à liquide de dialyse (9) du dialyseur (5) peut être traversée par le liquide de dialyse, où une pompe à sang (7) est branchée dans le circuit extracorporel et une pompe à liquide de dialyse (13) est branchée dans la voie de liquide de dialyse, avec

un dispositif (22) pour modifier la caractéristique physique ou chimique cdi du liquide de dialyse dans la voie de liquide de dialyse en amont du dialyseur (5) dans un intervalle de temps prédéterminé,

un dispositif de mesure (16) pour détecter la caractéristique physique ou chimique cdo du liquide de dialyse dans la voie de liquide de dialyse en aval du dialyseur (5),

une unité de mémoire (19) qui est agencée de telle manière que les valeurs mesurées de la caractéristique physique ou chimique cdo du liquide de dialyse en aval du dialyseur (5) peuvent être mémorisées tour à tour dans le temps et

une unité de calcul et d'évaluation (21) qui est agencée de telle manière que le paramètre de l'hémodialyse peut être déterminé d'après la caractéristique cdi et la caractéristique cdo,

**caractérisé en ce que** l'unité de calcul et d'évaluation (21) est agencée de telle manière que la réponse du dialyseur à une impulsion de Dirac comme signal d'entrée (réponse impulsionnelle) peut être déterminée d'après l'évolution temporelle de la caractéristique physique ou chimique du liquide de dialyse en amont et d'après l'évolution temporelle de la caractéristique physique ou chimique du liquide de dialyse en aval du dialsyeur (5), et le paramètre de l'hémodialyse est déterminé d'après la réponse impulsionnelle du dialyseur.

9. Dispositif selon la revendication 8 **caractérisé en ce que** l'unité de calcul et d'évaluation (21) comprend un intégrateur pour déterminer l'intégrale de la réponse impulsionnelle du dialyseur et une mémoire dans laquelle sont déposées des valeurs prédéterminées qui sont caractéristiques d'une dialysance déterminée et **en ce que** l'unité de calcul et d'évaluation est agencée de telle manière que la dialysance peut être déterminée comme paramètre hémodynamique par la comparaison de l'intégrale de la réponse impulsionnelle avec les valeurs déposées dans la mémoire.

10. Dispositif selon la revendication 8 **caractérisé en ce que** les valeurs, correspondant à un état stationnaire, de la caractéristique physique ou chimique $cdi_{stat}$ du liquide de dialyse en amont du dialyseur peuvent être déterminées d'après la réponse impulsionnelle du dialyseur (5) et d'après les valeurs de la caractéristique physique ou chimique cdi du liquide de dialyse en amont du dialyseur, et le paramètre de l'hémodialyse est déterminé d'après les valeurs, correspondant à un état stationnaire, de la caractéristique $cdi_{stat}$ et d'après les valeurs mémorisées de la caractéristique physique ou chimique cdo du liquide de dialyse en aval du dialyseur.

11. Dispositif selon l'une des revendications 8 à 10 **caractérisé en ce que** le dispositif (22) pour modifier la caractéristique physique ou chimique est agencé comme un dispositif pour modifier la concentration de substance dans le liquide de dialyse en amont du dialyseur (concentration d'entrée du liquide de dialyse cdi) ou la température du liquide de dialyse, et le dispositif de mesure (16) pour détecter la caractéristique physique ou chimique est agencé comme un dispositif de mesure pour détecter la concentration de substance dans le liquide de dialyse en aval du dialyseur (concentration de sortie du liquide de dialyse cdo) ou la température du liquide de dialyse.

12. Dispositif selon la revendication 11 **caractérisé en ce que** le dispositif de mesure (16) pour détecter la propriété physique ou chimique comporte un capteur de conductibilité ou capteur optique disposé dans la voie de liquide de dialyse en aval du dialyseur pour déterminer la concentration de sortie du liquide de dialyse cdo ou un capteur de température pour déterminer la température du liquide de dialyse.

Fig. 1

EP 0 894 013 B1

Fig. 2a

Fig. 2b